# EUROPÄISCHE PATENTSCHRIFT

(11) **EP 0 664 732 B1**
(45) Veröffentlichungstag und Bekanntmachung des Hinweises auf die Patenterteilung: **03.12.1997**
(21) Anmeldenummer: 93921903.6
(22) Anmeldetag: 02.10.1993
(51) Int. Cl.: B01J 31/08, B01J 31/10

(54) **TRÄGERKATALYSATOR UND VERWENDUNG DESSELBEN**
SUPPORT CATALYST AND USE OF SAME
CATALYSEUR A SUPPORT ET SON UTILISATION

(30) Priorität: 15.10.1992 DE 4234779
(43) Veröffentlichungstag der Anmeldung: 02.08.1995
(73) Patentinhaber: VEBA OEL AG, 45876 Gelsenkirchen (DE)
(72) Erfinder: HOFFMANN, Ulrich, D-37154 Northeim (DE); KUNZ, Ulrich, D-38678 Clausthal (DE); BRUDERRECK, Hartmut, D-46325 Borken (DE); GOTTLIEB, Klaus, D-58313 Herdecke (DE); SCHÄDLICH, Kuno, D-45136 Essen (DE); BECKER, Stefan, D-44789 Bochum (DE)
(74) Vertreter: Berg, Dirk, Dr.
(86) Internationale Anmeldenummer: EP9302696
(87) Internationale Veröffentlichungsnummer: WO9408713

(56) Entgegenhaltungen:
- EP-A- 0 065 925
- EP-A- 0 310 843
- EP-A- 0 417 407
- EP-A- 0 486 277
- EP-A- 0 492 064
- EP-A- 0 503 688
- EP-A- 0 541 267

## Beschreibung

Die Erfindung betrifft einen Trägerkatalysator und die Verwendung desselben.

Die Entwicklung neuer Trägerkatalysatoren, welche Sulfonsäuregruppen an einem Polymergerüst enthalten, ist für eine Reihe industriell ausgeübter chemischer Verfahren, beispielsweise der Herstellung gewisser Ether aus der Umsetzung von C₄- oder C₅-Fraktionen der Raffinerietechnik mit Methanol oder Ethanol als Zumisch-Komponenten für die Kraftstoffherstellung (vergl. Hydrocarbon Processing, November 1990, 126, 128) von großer Bedeutung.

Für die Herstellung verbesserter, insbesondere umweltverträglicherer klopffester Benzinqualitäten besonders wichtige Zumischkomponenten sind beispielsweise Methyl-tert.-butylether (MTBE) und tert.-Amylmethylether (TAME) bzw. tert.-Butylethylether (ETBE).

Die Verfahren zur Herstellung dieser Komponenten werden mit Vorteil als Reaktivdestillationsverfahren oder Verfahren zur Behandlung bzw. Umwandlung der Einsatzprodukte mittels katalytischer Destillation ausgeführt. Die Reaktion und die sich üblicherweise in einem weiteren Verfahrensschritt anschließende Aufarbeitung des Reaktionsgemisches durch Destillation bzw. Rektifikation erfolgen bei der katalytischen Destillation in nur noch einem einzigen Reaktionsapparat, der den Aufarbeitungsteil mit einschließt.

Hierbei stellt die Einbringung und Fixierung des Katalysators in den Reaktionsapparat ein besonderes Problem dar. Üblicherweise wird bei festen oder Trägerkatalysatoren eine Schüttung des Katalysators in einem oder mehreren übereinanderliegenden Festbetten, die von dem Reaktionsgemisch durchströmt werden, vorgesehen.

Wenn die bei der industriellen Herstellung von MTBE oder TAME verwendete makroporöse vernetzte Polystyrolsulfonsäure in Kugelform als lose Schüttung zu einem Festbett als Katalysator bei der Umsetzung von Methanol und Buten eingesetzt wird so sind damit verschiedene Nachteile, wie hoher Strömungswiderstand, Randgängigkeit sowie Katalysatorabrieb verbunden.

Zur Vermeidung dieser Nachteile ist bei einem Verfahren zur Durchführung chemischer Reaktionen in einem Reaktions-Destillationssapparat vorgeschlagen worden, den aus makroporösen vernetzten, gelförmigen Körnern einer Polystyrolsulfonsäure bestehenden Katalysator in geschlossenen Taschen unterzubringen, die aus Drahtgewebe gebildet und gehalten sowie durch Aufwickeln in Form einer Spirale zu einem dem Reaktionsapparat angepaßten Festbett angeordnet werden, vgl. EP-A-O 466 954.

Es ist ferner vorgeschlagen worden, zur Verbesserung der Stoffausstauscheigenschaften der Festbettkatalysatorschüttung, dem Katalysator die Form von Austauschkörpern wie Raschig-Ringen zu geben, wozu eine Mischung aus einem vernetzten Styroldivinylbenzolcopolymer in gemahlener Form mit dem Thermoplasten Polypropylen zu derartigen Austauschkörpern koextrudiert und anschließend sulfoniert wird, vgl. FR-A-2 297 083.

Dieses Konzept ist dahingehend weiterentwickelt worden, daß Formkörper aus makroporösen Ionenaustauscherharzen ohne Mitverwendung eines Thermoplasten direkt hergestellt wurden, vgl. DE-A-3 930 515. Die hiernach erhaltenen Formkörper weisen die erwarteten guten Stoffaustauscheigenschaften sowie eine gute katalytische Aktivität auf. Sie sind aber nicht leicht in industriellen Mengen herzustellen und lassen hinsichtlich ihrer mechanischen Festigkeit zu wünschen übrig.

Aus der US 4 250 052 sind Trägerkatalysatoren in Form von Füllkörpern bekannt, die mit einem Polymer aus vinylaromatischen Monomeren beschichtet und anschließend sulfoniert werden können. Dazu wird das Polymere gelöst, auf den Träger aufgebracht und das Lösungsmittel wieder entfernt. Ein Nachteil dieses Verfahrens bzw. der so hergestellten Katalysatoren ist darin zu sehen, daß die genannten Polymeren nicht stark vernetzt sein können, weil sie sonst nicht in Lösung zu bringen sind. Dies hat zur Folge, daß sie auch durch das Reaktionsgemisch an-, ab- oder aufgelöst werden können. Außerdem läßt sich auf die genannte Weise keine Makroporosität des Polymeren erreichen.

Die Aufgabe der vorliegenden Erfindung besteht nun darin, einen Trägerkatalysator mit Stoffaustauscheigenschaften, der auch leicht in industriellen Mengen herstellbar ist zur Verfügung zu stellen.

Die Aufgabe wird durch Trägerkatalysatoren gemäß den Ansprüchen 1 bis 14 sowie durch die Verwendung dieser Trägerkatalysatoren gemäß den Ansprüchen 15 bis 24 gelöst.

EP-A-0 310 843 beschreibt einen sauren Katalysator vom Typ eines H⁺-Ionenaustauschers mit katalytisch wirksamen Säuregruppen. Die ionenaustauschaktiven Gruppen, insbesondere Sulfongruppen, werden direkt über organische Silane an die äußere Oberfläche des Trägermaterials gebunden.

EP-A-0 503 688 offenbart ein Verfahren zum Aufbringen eines Polymeren auf ein Trägermaterial durch Tränken des Trägermaterials in einer Lösung oder Suspension des Polymeren, Entfernen des Lösungsmittels und Erhitzen des so beschichteten Trägermaterials über die Glastemperatur des Polymeren.

EP-A-0 065 925 beschreibt ein katalytisch wirksames Austauscherharz, welches hergestellt werden kann durch Tränken eines porösen mineralischen Trägermaterials mit den Monomeren und anschließender Polymerisation. Vor der Polymerisation wird jedoch das Lösungsmittel entfernt.

EP-A-0 541 267 offenbart ein Verfahren zur Herstellung von Alkyl-tert.-alkylethern in Gegenwart eines Katalysators, der aus einem auf ein inertes Trägermaterial aufgebrachten, Sulfonsäuregruppen enthaltenden Fluorkohlenstoff besteht.

Die Trägerkatalysatoren gemäß der Erfindung in Gestalt von Füllkörpern sind aufgebaut aus einem Grundkorper aus offenporigem Tragermaterial, auf dessen äußerer und innerer Oberfläche ein Makroporen aufweisendes Ionenaustauscherharz mechanisch und/oder chemisch verankert ist.

Eine chemische Verankerung kann bevorzugt sein und ist beispielsweise durch eine Silanisierung der Oberfläche des offenporigen Trägermaterials mit anschließender Aufbaupolymerisation zu erhalten.

Die Füllkörper des Trägerkatalysators sind als Raschig-Ringe, Berl-Sättel, Torus-Sättel, Füllringe mit Steg oder Kreuzsteg, Pall-Ringe, sonstige Hohlkörper, Hohlkugeln, geordnete Packungen, Wabenkörper u. dgl. mit einem Hohlraumanteil des makroporösen Ionenaustauscherharzes von 5 bis 95 Vol.-% ausgebildet.

Das Trägermaterial des vorgenannten Trägerkatalysators besteht aus offenporigem Glas, Sinterglas, offenporigem Keramikmaterial auf Aluminiumsilicatbasis, Sinterglas-Keramik, Schaumkeramik, Aktivkohle oder Aktivkoks.

Bei Sinterglas bzw. Sinterglas-Keramik kann durch eine vorherige Behandlung mit wäßriger Alkalihydroxidlösung die Oberfläche vergrößert werden. Einerseits entstehen dadurch vermehrt Silanolgruppen auf der Oberfläche, die dann einer Silanisierung zugänglich sind und andererseits entsteht durch den Ätzvorgang eine stärkere Rauhigkeit der Oberfläche, die eine mechanische Fixierung der aufgebrachten Polymeren begünstigt.

Während offenporiges Glas, Sinterglas, Sinterglas-Keramik oder offenporiges Keramikmaterial bereits in Form für Stoffaustauschzwecke geeigneter Grund- bzw. Füllkörper im Handel erhältlich sind, können Aktivkohle oder Aktivkoks mit geeigneten Porengrößen unter Auswahl geeigneter Absiebungen in ausgewählten Stückgrößenbereichen in Form loser Schüttungen zu Katalysatorbetten zur erfindungsgemäßen Anwendung kommen.

Das Makroporen aufweisende Ionenaustauscherharz auf und in dem Trägermaterial ist vorzugsweise eine vernetzte Polystyrolsulfonsäure, wobei durch Verwendung von mehr oder weniger Divinylbenzol oder Diisopropenylbenzol ein unterschiedlicher Vernetzungsgrad entsprechend den Erfordernissen vorgegeben werden kann. Vorzugsweise ist das Polymere derartig hoch vernetzt, daß es nicht durch das Reaktionsgemisch der zu katalysierenden Reaktion herausgelost werden kann.

Das Ionenaustauscherharz kann durch zwei unterschiedliche Verfahren auf das Trägermaterial aufgebracht werden.

Bei dem sogenannten Tränkpolymerisationsverfahren werden die Formkörper mit der Reaktionsmischung getränkt, nicht aufgenommene Tränklösung wird entfernt und anschließend wird die Polymerisation durchgeführt.

Bei dem Fällungspolymerisationsverfahren liegen die Füllkörper während der Polymerisation in einem Überschuß an Reaktionsmischung. Die Vorteile des Fällungspolymerisationsverfahrens sind eine sehr gleichmäßige Verteilung des Polymeren im Porenraum des Trägermaterials, außerdem eine hohe Porosität, da das Lösungsmittel als Porenbildner wirken kann, sowie eine einfache Herstellungsweise, weil Maßnahmen zur Vergleichmäßigung der Verteilung der Monomeren auf dem Träger nicht nötig sind.

Desweiteren ist von Vorteil, daß der Gehalt an Polymeren im fertigen Ionenaustauscherharz einfach durch das Verhältnis eines geeigneten Lösungsmittels zu der Monomermischung eingestellt werden kann und daß das Polymer bei Einsatz des entsprechenden Lösungsmittels schon in gequollener Form entsteht, so daß eine mögliche Schädigung des Trägermaterials durch Quellungsvorgänge weitgehend vermieden werden kann. Als Lösungsmittel können z. B. Methanol oder i-Octan sowie Pentadecan verwendet werden. Auch ein C₁₄-bis C₁₇-n-Paraffin-Schnitt ist verwendbar.

Durch die genannten Verfahren wird im Porenraum und auf der Oberfläche eines porösen Füllkörpers ein Makroporen aufweisendes Polymer erzeugt, dem anschließend durch Sulfonierung Ionenaustauschereigenschaften verliehen werden.

Beim Fällungspolymerisationsverfahren erfolgt die Polymerisation je nach Konzentration der Ausgangsstoffe Styrol und Divinylbenzol bzw. Diisopropylbenzol in dem Porenbildner oder auch dem Lösungsmittel im Porenraum des Füllkörpers bis zu dem Polymerisationsgrad, bei dem das gebildete Polymere in flockiger Form in dem Porenbildner bzw. dem Lösungsmittel unlöslich wird und ausfällt. Die hierdurch im Porenraum des Trägermaterials erzeugten Polymerflocken können in den Trägermaterialporen rein mechanisch fixiert sein und sind durch das umgebende Trägermaterial vor mechanischen Beschädigungen geschützt.

Für bestimmte Reaktionen ist es zweckmäßig, Trägerkatalysatoren der vorstehend beschriebenen Art mit Metallen der 7. oder auch 8. Nebengruppe des Periodensystems, insbesondere Palladium, Platin, Ruthenium oder Rhodium in Mengen von 0,1 bis 100 g/kg Ionenaustauscherharz zu dotieren.

Wie bereits angegeben, kann das Polymere zusätzlich chemisch am Trägermaterial fixiert sein. Hierzu wird ein geeigneter chemischer Koppler verwendet. Bei allen Formkörpern, die OH-Gruppen an ihrer Oberfläche aufweisen, sind z. B. Silane als derartige Koppler geeignet.

Basiert das anzukoppelnde Polymere auf vinylischen Monomeren, so bieten sich vorzugsweise Vinylgruppen tragende Silane als Koppler an, insbesondere Phenylvinyldiethoxysilan, Phenylmethylvinylsilan, Trieethoxyvinylsilan oder Trichlorvinylsilan.

Der Trägerkatalysator kann hergestellt werden durch Tränken der Füllkörper mit 0,1 bis 60 Gew.-% einer Mischung aus 10 bis 80, vorzugsweise 30 bis 70 Gew.-% Styrol, 2 bis 25, vorzugsweise 5 bis 10 Gew.-% Divinylbenzol, 1 bis 88, vorzugsweise 20 bis 50 Gew.-% eines Porenbildners bzw. eines Lösungsmittels sowie einer wirksamen Menge eines Polymerisationsinitiators, Durchführung der Polymerisationsreaktion unter Temperaturerhöhung auf 30 bis 90 °C und anschließende Behandlung des in den Poren des Füllkörpers verankerten polymeren Materials mit einer sulfonierenden Säure. Hierbei ist der Gewichtsanteil des Porenbildners bzw. des Lösungsmittels so zu bemessen, daß die Gewichtsanteile der Mischung aus Styrol und Divinylbenzol zu 100 Gew.-% ergänzt werden.

Ein Trägerkatalysator hergestellt nach dem Fällungspolymerisationsverfahren ist erhältlich durch Hinzugeben einer Mischung im Gewichtsverhältnis von 10 zu 1 bis 1 zu 10 aus 10 bis 80, vorzugsweise 20 bis 50 Gew.-% Styrol, 2 bis 25, vorzugsweise 5 bis 10 Gew.-% Divinylbenzol, 1 bis 88, vorzugsweise 20 bis 50 Gew.-% eines Porenbildners bzw. Lösungsmittels sowie einer wirksamen Menge eines Polymerisationsinitiators einerseits und einer C₁₄-bis C₁₇-n-Paraffinfraktion andererseits zu 5 bis 50 Gew.-% eines auf die Gesamtmischung bezogenen Anteils des Trägermaterials, Konditionieren unter Vakuum, Auspolymerisierenlassen, Auswaschen von überschüssigem Porenbildner und äußerlich anhaftendem Polymergel und anschließende Behandlung mit einer sulfonierenden Säure.

Als Porenbildner können C₆- bis C₁₆-Alkane, z. B. n-Heptan, Pentadecan, i-Octan, sowie die C₉- bis C₁₃-Fraktionen der n-Paraffinherstellung eingesetzt werden. Diese Porenbildner haben eine gute Löslichkeit für die zur Herstellung des Ionenaustauscherharzes eingesetzten Monomeren Styrol und Divinylbenzol, jedoch nur ein geringes Quellvermögen für das entstehende Polymer (vgl. Catalytic Chemistry, Bruce, C. Gates, John Wiley & Sons, 1992, S. 221).

Auf diese Weise bildet sich in den Hohlräumen des Trägers und auf dem Trägermaterial eine vorzugsweise aus Mikrospheren bestehende Festphase und die ursprünglich von dem Lösungsmittel für die Monomeren eingenommenen Volumina verbleiben nach Entfernen des Lösungsmittels als Makroporen, die das gesamte vernetzte Polystyrol durchziehen und so einen guten Zugang der Reaktanden für die beabsichtigten chemischen Reaktionen nach Funktionalisierung mit der sulfonierenden Säure ermöglichen. Gleichzeitig kann dadurch eine Erhöhung der aktiven Oberfläche erreicht werden.

In einer bevorzugten Ausführungsform können daher die Porenbildner auch Lösungsmittel für die Monomermischung bei der Fällungspolymerisation sein. Bei der Fällungspolymerisation sind auch niedere Alkanole wie Methanol als Lösungsmittel geeignet. Auch Kombinationen von Lösungsmittel und Porenbildner können engesetzt werden.

Die sulfonierende Säure kann eine aromatische oder eine aliphatische Sulfonsäure, Chlorsulfonsäure oder Schwefelsäure sein. Desweiteren sind Schwefeldioxid sowie Schwefeldioxidadditionsverbindungen wie die des Dioxans, des Dimethylanilins oder des Pyridins geeignet.

Schwefelsäure ist weniger bevorzugt, da sie in den für eine ausreichende Sulfonierung erforderlichen Konzentrationen bereits eine merkliche oxidative Wirkung zeigt und zu einem Abbau des Polymergerüstes führen kann.

Bevorzugt sind als aromatische Sulfonsäure Benzolsulfonsäure und als Vertreter einer aliphatischen Sulfonsäure Methylsulfonsäure.

In einer weiteren bevorzugten Ausführungsform können die Säuren auch Lösungsmittel wie Chloroform, Nitromethan oder Acetonitril enthalten.

Die erfindungsgemäßen Trägerkatalysatoren werden mit Vorteil zur Durchführung der chemischen Reaktion der Veretherung, Veresterung, Hydrierung, Alkylierung, Hydratisierung, Dimerisierung, Oligomerisierung oder von Kombinationen derselben sowie zu den jeweiligen Umkehrreaktionen eingesetzt.

Besonders bevorzugt ist die Durchführung einer der oben genannten chemischen Reaktionen mit gleichzeitig angewandter Trennoperation wie Adsorption, Absorption, Extraktion, Strippen, Destillation, Rektifikation, Fraktionierung, Membranverfahren oder dgl. zur Abtrennung der gewünschten Produkte. Hierbei ist die Gegenstromführung von Gas- und Flüssigphase für ein- und mehrphasige Gas- und Flüssigreaktionen geeignet, da die erfindungsgemäßen Trägerkatalysatoren einen hohen Lückengrad aufweisen und somit einen niedrigen Druckverlust verursachen.

Eine bevorzugte chemische Reaktion unter Verwendung des erfindungsgemäßen Trägerkatalysators ist die chemische Reaktion der Veretherung und die Trennung der Reaktionsprodukte durch reaktive Destillation zur Gewinnung von tert. Alkylethern aus der Umsetzung von Alkanolen mit Alkenen, insbesondere von MTBE aus der Umsetzung von Methanol mit i-Buten, zur Gewinnung von i-Propyl-tert.-butylehter (PTBE) aus der Umsetzung von i-Propanol mit i-Buten, zur Gewinnung von ETBE aus der Umsetzung von i-Buten mit Ethanol oder zur Gewinnung von TAME aus der Umsetzung von i-Penten-(1) oder i-Penten-(2) mit Methanol. Weitere bevorzugte Reaktionen sind die Herstellung von i-Propanol aus der Umsetzung von Propen mit Wasser und die Herstellung von tert.-Butylalkohol (TBA) durch Umsetzung von i-Buten mit Wasser.

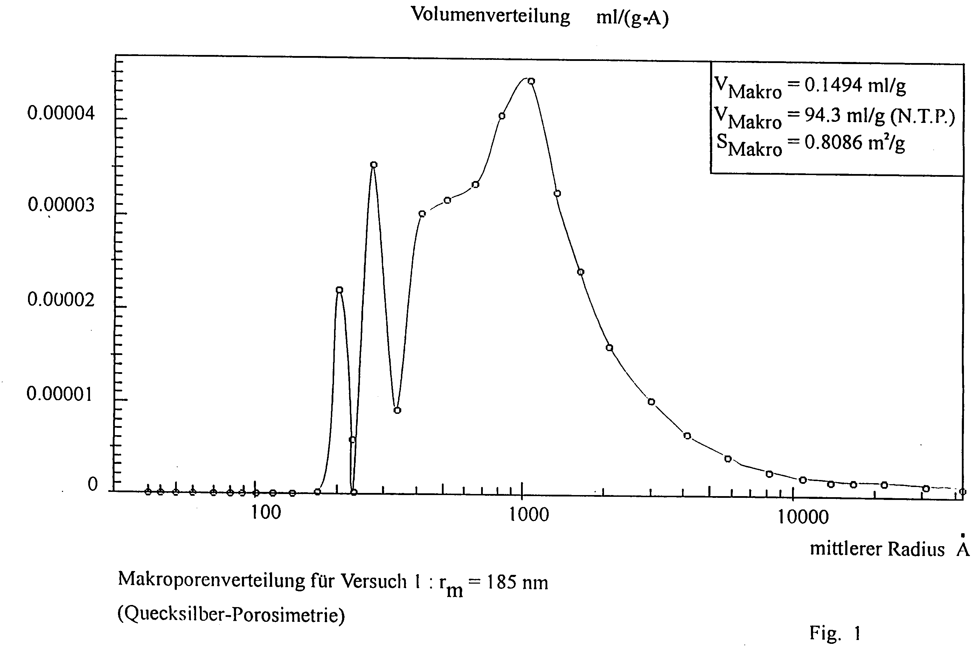

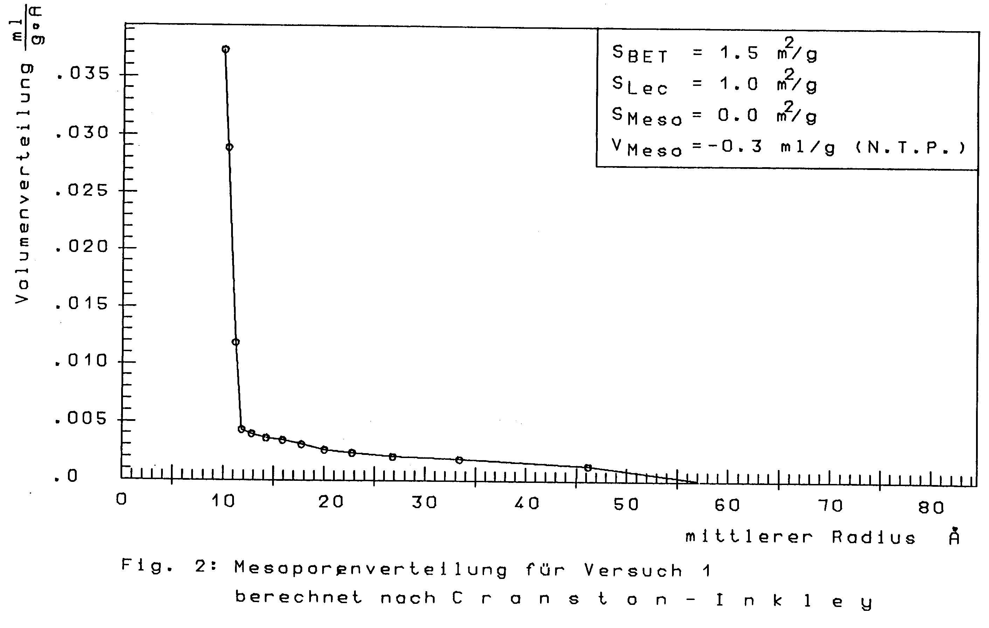

Die vorliegende Erfindung wird anhand der nachstehenden Beispiele sowie der in den Tabellen 1 und 2 und den Figuren 1 und 2 mitgeteilten Ergebnisse weiter ins Einzelne gehend erläutert.

### Beispiel 1

Als Trägermaterial wurden Formkörper aus offenporigem Sinterglas in der Form von Raschig-Ringen in den Abmessungen 8,8 mm : 9 mm (Außendurchmesser x Höhe) eingesetzt.

Dieses Trägermaterial zeichnet sich durch eine Oberfläche bis 0,4 m²/g und ein Porenvolumen bis 70 % aus. Der Porendurchmesser ist von 1,6 µm bis 400 µm variierbar, die Temperaturbeständigkeit reicht bis 450 °C.

40 Stück der oben genannten Ringe wiesen eine Masse von 12,5 g auf und wurden mit einer Mischung aus 22,7 g Styrol, 13,7 g Pentadecan, 2,9 g Divinylbenzol und 50 mg Azoisobutyronitril getränkt. Nicht im Porenraum aufgenommene Tränklösung wurde entfernt. Die getränkten Ringe wurden in einen druckdicht schließenden Metallbehälter gebracht und bei einer Temperatur von ca. 75 °C im Wärmeschrank im Verlauf von 10 h polymerisiert. Der druckdichte Behälter ist erforderlich, damit sich die Monomermischung während der Polymerisation nicht durch Verdampfungsvorgänge verändert. Der Polymergehalt der so behandelten Ringe lag bei ca. 20 bis 25 Gew.-%. Dann wurden die Ringe auf Raumtemperatur abgekühlt und der Sulfonierung unterzogen. 500 ml der erhaltenen Formkörper wurden vollständig mit Chloroform bedeckt und mit 50 ml Chlorsulfonsäure unter Ausschluß von Feuchtigkeit ca. 20 Stunden zur Reaktion gebracht. Anschließend wurde die Reaktionslösung langsam auf Eis gegossen, die Formkörper wurden mit Chloroform gespült und mit Methanol sowie mit Deionat sulfonierungsmittel- und säurefrei gespült. Die Formkörper wurden in Wasser gelagert.

### Beispiel 2

12,5 g des in Beispiel 1 beschriebenen Trägermaterials wurden zu einer Mischung im Gewichtsvernältnis von 1 zu 1 aus 22,7 g Styrol, 2,9 g Divinylbenzol, 13,8 g i-Oktan, 0,05 g Azoisobutyronitril einerseits und C₁₄ bis C₁₇-n-Paraffin-Fraktion andererseits hinzugegeben, so daß die zu imprägnierenden Formkörper vollständig bedeckt waren. Dann wurde die Mischung zwei min unter Vakuum konditioniert, um sämtliche Poren zu füllen. Anschließend wurde die Mischung 16 h bei 60 °C erwärmt.

Das die Formkörper umgebende Polymergel sowie der Porenbildner wurden nach beendeter Reaktion mit Chloroform abgewaschen. Die so hergestellten Formkörper enthielten ca. 10 Gew.-% Polymer.

Durch Wiederholen der Behandlung nach dieser Methode kann der Polymergehalt bis auf etwa 20 Gew.-% gesteigert werden.

500 ml der erhaltenden Formkörper wurden vollständig mit Chloroform bedeckt und mit 50 ml Chlorsulfonsäure unter Ausschluß von Feuchtigkeit ca. 20 Stunden zur Reaktion gebracht. Anschließend wurde die Reaktionslösung langsam auf Eis gegossen, die Formkörper wurden mit Chloroform gespült und mit Methanol sowie mit Deionat sulfonierungsmittel- und saurefrei gespült. Die Formkörper wurden mit Wasser gelagert.

Die in Tabelle 1 benutzten Abkürzungen besitzen folgende Bedeutung:
- Kap.:: Kapazität des eingesetzten Katalysators
- nᵢ:: Molenstrom der Komponente i
- t:: Verweilzeit
- Xᵢ:: Umsatz der Komponente i
- Yᵢ:: Ausbeute an MTBE bezogen auf die Komponente i
- Komponente i:: MeOH bzw. IB
- MeOH:: Methanol
- IB:: i-Buten
- sSDCmT:: Sulfoniertes Styrol/Divinyl-Benzol-Copolymer, Makroporen aufweisend, durch Tränkpolymerisation hergestellt
- sSDCmF:: Sulfoniertes Styrol/Divinyl-Benzol-Copolymer, Makroporen aufweisend, durch Fällungspolymerisation hergestellt
- sSDC:: Sulfoniertes Styrol/Divinyl-Copolymer
- MPI:: Makroporöse Ionenaustauscherringe

In Tabelle 2 sind die effektiven Reaktionsgeschwindigkeiten der MTBE-Bildung angegeben. Sie beziehen sich zum einen auf die Kapazität des Katalysators, zum anderen auf die Masse und schließlich auf das Schüttvolumen des trockenen Katalysators.

Die Schüttdichte S des trockenen Katalysators wurde durch Wägung der Masse, die ein gegebenes Schüttvolumen einnimmt, oder durch Messen des Volumens einer gegebenen Masse ermittelt. In dem vorliegenden Fall wurde ein Volumen vorgegeben.

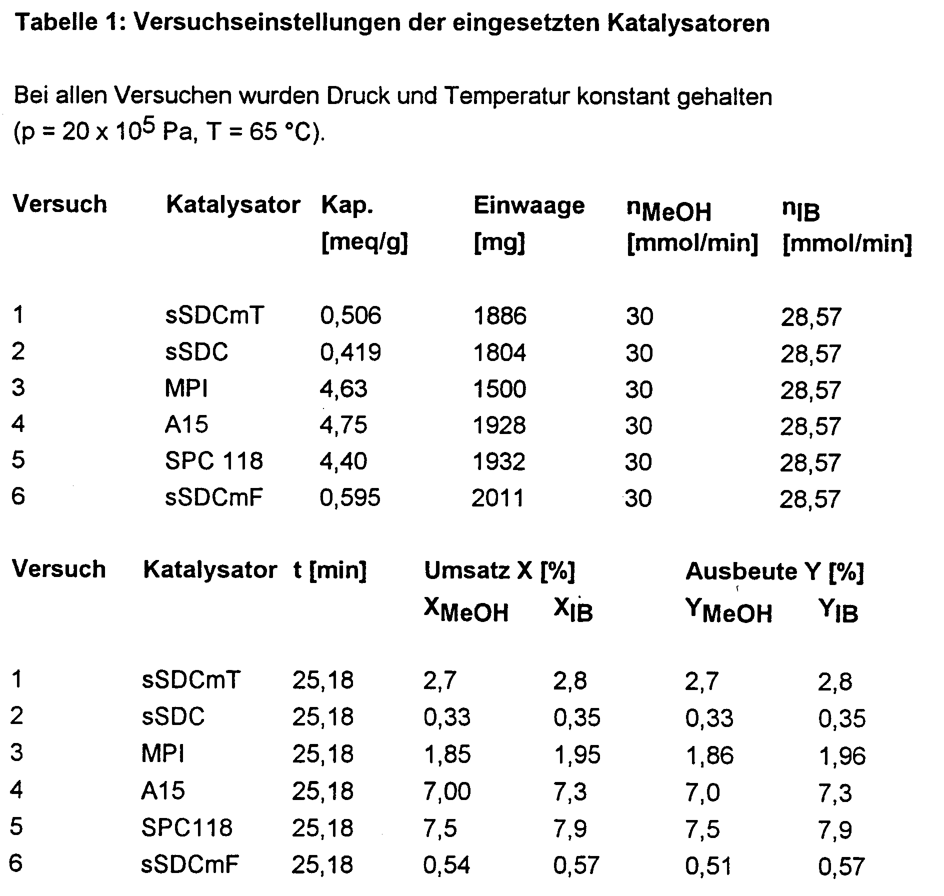

**Tabelle 2**

| **Ergebnisse der eingesetzten Katalysatoren** | | | | | |
|---|---|---|---|---|---|
| **Versuch** | **Kat.** | **ρ [g/ml]** | **Rate e**_{**eff**} **(MTBE) [mmol/(s eq)]** | **Rate m**_{**eff**} **(MTBE) [mmol/(s g)]x10**^{**3**} | **Rate V**_{**eff**} **(MTBE) [mmol/(s ml)]x10**^{**3**} |
| 1 | sSDCmT | 0,328 | 14,1 | 7,13 | 2,34 |
| 2 | sSDC | 0,328 | 2,24 | 0,94 | 0,31 |
| 3 | MPI | 0,384 | 1,34 | 6,20 | 2,38 |
| 4 | A15 | 0,577 | 3,82 | 18,14 | 10,46 |
| 5 | SPC118 | 0,506 | 4,43 | 19,49 | 9,86 |
| 6 | sSDCmF | 0,328 | 2,27 | 1,35 | 0,44 |
| - Rate Z_{eff, MTBE}: Reaktionsgeschwindigkeit der MTBE-Bildung - mit Z = e: bezogen auf Equivalente des eingesetzten Harzes - mit Z = m: bezogen auf Masse des eingesetzten Harzes - mit Z = V: bezogen auf Schüttvolumen des eingesetzten Harzes | | | | | |

Der Katalysator aus Versuch 1 wurde gemäß dem Beispiel 1 hergestellt.

Der Katalysator aus Versuch 2 wurde analog hergestellt, jedoch ohne Porenbildner und weist somit keine Makroporen auf. Dieser Katalysator ist in der Testreaktion der MTBE-Bildung deutlich schlechter als der aus Versuch 1.

Der Katalysator aus Versuch 6 wurde gemäß dem Beispiel 2 hergestellt. In der Aktivität liegt er zwar unter der des Katalysators aus Versuch 1, ist aber in der Herstellung weniger aufwendig. Die Versuche 3 bis 5 sind Vergleichsversuche mit Standardionenaustauschern. A15 ist ein Amberlyst der Fa. Rohm & Haas und SPC118 ein Produkt der Fa. Bayer. Die Polymer-Ringe mit der Bezeichnung MPI sind in dem Dokument EP 0 417 407 A1 beschrieben.

Die nachfolgenden Fig. 1 und 2 der Zeichnung zeigen die Makro- und Mesoporen-Verteilung des Katalysators gemäß Versuch 1.

Die Abkürzungen bedeuten:
Fig 1:
   - V_{Makro}=: Volumen der Makroporen in ml/g gemessen mit Quecksilberporosimetrie
   - V_{Makro}=: Volumen an adsorbiertem Helium in den Makroporen in ml/g [N.T.P.] gemessen durch Heliumadsorption
   - S_{Makro}=: Oberfläche der Makroporen in m²/g
   - rₘ =: Mittlerer Porenradius (hier der Makroporen)
Fig. 2:
   - S_{BET} =: Spezifische Oberfläche der Probe in m²/g gemessen nach der BET-Methode
   - S_{Lec} =: Oberfläche anhand der Standardisotherme nach Lecloux
   - S_{Meso} =: Oberfläche der Mesoporen in m²/g
   - V_{Meso} =: Volumen der Mesoporen

Nach IUPAC (1972) sind die Durchmesser von
- Mikroporen < 2 nm
- Mesoporen 2 nm bis 50 nm
- Makroporen > 50 nm.

## Patentansprüche

1. Trägerkatalysator in Gestalt von Füllkörpern und aufgebaut aus einem offenporigen Trägermaterial, auf dessen äußerer und innerer Oberfläche sowie im Porenraum ein Makroporen aufweisendes Ionenaustauscherharz mechanisch und/oder chemisch verankert ist, herstellbar durch Tränken oder vollständiges Bedecken des als Füllkörper ausgebildeten Trägermaterials mit einer Mischung aus polymerisierbaren Monomeren sowie Lösungsmittel und/oder Porenbilder, Durchführung der Polymerisationsreaktion und ggfs. Funktionalisierung zur Einführung ionenaustauschaktiver Gruppen.

2. Trägerkatalysator nach Anspruch 1, dadurch gekennzeichnet, daß die Füllkörper als Raschig-Ringe, Berl-Sättel, Torus-Sättel, Füllringe mit Steg oder Kreuzsteg, Pall-Ringe, sonstige Hohlkörper, Hohlkugeln, geordnete Packungen, Wabenkörper mit einem Hohlraumanteil des makroporösen Ionenaustauscherharzes von 5 bis 95 Vol.-% ausgebildet sind.

3. Trägerkatalysator nach Anspruch 1 oder 2, dadurch gekennzeichnet, daß das Trägermaterial aus offenporigem Glas, Sinterglas, offenporigem Keramikmaterial auf Aluminiumsilikatbasis, Sinterglas-Keramik, Schaumkeramik, Aktivkohle oder Aktivkoks besteht.

4. Trägerkatalysator nach einem der Ansprüche 1 bis 3, dadurch gekennzeichnet, daß das an seiner Oberfläche OH-Gruppen tragende offenporige Trägermaterial mit einem Vinylgruppen tragenden Silan als Koppler umgesetzt wird an welchem durch nachfolgende Sulfonierung saure Ionenaustauschergruppen fixiert sind.

5. Trägerkatalysator nach einem der Ansprüche 1 bis 4, dadurch gekennzeichnet, daß das makroporöse Ionenaustauscherharz eine makroporöse vernetzte Polystyrolsulfonsäure ist.

6. Trägerkatalysator nach einem der Ansprüche 1 bis 5 dotiert mit Metallen der 7. oder auch 8. Nebengruppe des Periodensystems, insbesondere Palladium, Platin, Ruthenium, Rhodium, in Mengen von 0,1 bis 100 g/kg Ionenaustauscherharz.

7. Trägerkatalysator nach einem der Ansprüche 1 bis 6, erhältlich durch Tränken der Füllkörper mit 0,1 bis 60 Gew.-% einer Mischung aus 10 bis 80, vorzugsweise 30 bis 70 Gew.-% Styrol, 2 bis 25, vorzugsweise 5 bis 10 Gew.-% Divinylbenzol, 1 bis 88, vorzugsweise 20 bis 50 Gew.-% eines Porenbildners bzw. eines Lösungsmittels sowie einer wirksamen Menge eines Polymerisationsinitiators, Durchführung der Polymerisationsreaktion unter Temperaturerhöhung auf 30 bis 90 °C und anschließende Behandlung mit einer sulfonierenden Säure.

8. Trägerkatalysator nach einem der Ansprüche 1 bis 6, erhältlich durch Hinzugeben einer Mischung aus (A) 10 bis 80, vorzugsweise 20 bis 50 Gew.-% Styrol, 2 bis 25, vorzugsweise 5 bis 10 Gew.-% Divinylbenzol, 1 bis 88, vorzugsweise 20 bis 50 Gew.-% eines Porenbildners bzw. Lösungsmittels sowie einer wirksamen Menge eines Polymerisationsinitiators und (B) einer C₁₄-bis C₁₇-n-Paraffinfraktion im Gewichtsverhältnis (A):(B) von 10:1 bis 1:10 zu 5 bis 50 Gew.-% eines auf die Gesamtmischung bezogenen Anteils des Trägermaterials, Konditionieren unter Vakuum, Auspolymerisierenlassen, Auswaschen von überschüssigem Porenbildner und äußerlich anhaftendem Polymergel und anschließende Behandlung mit einer sulfonierenden Säure.

9. Trägerkatalysator nach einem der Ansprüche 7 oder 8, dadurch gekennzeichnet, daß das Styrol teilweise durch Fluor-Styrol ersetzt wird.

10. Trägerkatalysator nach einem der Ansprüche 1 bis 9, dadurch gekennzeichnet, daß der Porenbildner eine C₆-bis C₁₆-Alkan, wie n-Heptan, Pentadecan, i-Octan oder eine hauptsächlich C₉- bis C₁₃-enthaltende n-Paraffinfraktion ist.

11. Trägerkatalysator nach einem der Ansprüche 1 bis 10, dadurch gekennzeichnet, daß das Lösungsmittel Methanol, i-Octan, Pentadecan oder ein C₁₄-bis C₁₇-n-Paraffin-Schnitt ist.

12. Trägerkatalysator nach einem der Ansprüche 1 bis 11, dadurch gekennzeichnet, daß die Funktionalisierung mittels einer sulfonierenden Säure, vorzugsweise einer aromatischen oder aliphatischen Sulfonsäure, Chlorsulfonsäure oder Schwefelsäure erfolgt.

13. Trägerkatalysator nach Anspruch 12, dadurch gekennzeichnet, daß die aromatische Sufonsäure Benzolsulfonsäure ist.

14. Trägerkatalysator nach Anspruch 12, dadurch gekennzeichnet, daß die aliphatische Sulfonsäure Methylsulfonsäure ist.

15. Verwendung eines Trägerkatalysators gemäß einem der Ansprüche 1 bis 14 zur Durchführung der chemischen Reaktion der Veretherung, Veresterung, Hydratisierung, Dimersisierung, Hydrierung, Alkylierung, Oligomerisierung oder Kombinationen derselben.

16. Verwendung nach Anspruch 15, gekennzeichnet durch gleichzeitig angewandte Trennoperationen wie Adsorption, Absorption, Extraktion, Strippen, Destillation, Rektifikation, Fraktionierung oder Membranverfahren.

17. Verwendung nach Anspruch 15 oder 16, gekennzeichnet durch Gegenstromführung von Gas- und Flüssigphase.

18. Verwendung nach einem der Ansprüche 15 bis 17, gekennzeichnet durch die Umsetzung von Alkanolen zu tert.-Alkylethern.

19. Verwendung nach einem der Ansprüche 15 bis 18, gekennzeichnet durch die Umsetzung von Methanol und i-Buten zu Methyl-tert.-butylether (MTBE).

20. Verwendung nach einem der Ansprüche 15 bis 18, gekennzeichnet durch die Umsetzung von i-Propanol und i-Buten zu i-Propyl-tert.-butylether (PTBE).

21. Verwendung nach einem der Ansprüche 15 bis 18, gekennzeichnet durch die Umsetzung i-Buten und Ethanol zu Ethyl-tert.-butylether (ETBE).

22. Verwendung nach einem der Ansprüche 15 bis 18, gekennzeichnet durch die Umsetzung von i-Penten-(1) oder i-Penten-(2) mit Methanol zu tert.-Amylmethylether (TAME).

23. Verwendung nach einem der Ansprüche 15 bis 17, gekennzeichnet durch die Umsetzung von Propen und Wasser zu i-Propanol.

24. Verwendung nach einem der Ansprüche 15 bis 17, gekennzeichnet durch die Umsetzung von i-Buten und Wasser zu tert.-Butylalkohol (TBA).

## Claims

1. Supported catalyst in the form of packing bodies and made up of an open-pore support material on whose outer and inner surface, and also in the pore space, an ion exchanger resin having macropores is mechanically and/or chemically anchored, capable of preparation by impregnation or complete coverage of the support material, formed as packing bodies, with a mixture of polymerizable monomers and solvents and/or pore forming agents, performance of the polymerization reaction and, if necessary, functionalization for the purpose of introducing ion-exchange-active groups.

2. Supported catalyst according to Claim 1, characterized in that the packing bodies are formed as Raschig rings, Berl saddles, torus saddles, packing rings with ridge or cross bridge, Pall rings, other hollow bodies, hollow spheres, ordered packings and honeycomb bodies having a hollow space component of the macroporous ion exchanger resin of 5 to 95% by volume.

3. Supported catalyst according to Claim 1 or 2, characterized in that the support material is composed of open-pole glass, sintered glass, open-pore ceramic material with an aluminium silicate base, sintered-glass ceramic, expanded ceramic, active carbon or active coke.

4. Supported catalyst according to one of Claims 1 to 3, characterized in that the open-pore support material carrying OH groups at its surface is reacted with a silane carrying vinyl groups as coupler to which acidic ion exchanger groups are fixed by subsequent sulphonation.

5. Supported catalyst according to one of Claims 1 to 4, characterized in that the macroporous ion exchanger resin is a macroporous crosslinked polystyrenesulphonic acid.

6. Supported catalyst according to one of Claims 1 to 5, doped with metals of group VB and VII of the periodic system, in particular palladium, platinum, ruthenium, rhodium, in amounts of 0.1 to 100 g/kg of ion exchanger resin.

7. Supported catalyst according to one of Claims 1 to 6, obtainable by impregnating the packing bodies with 0.1 to 60% by weight of a mixture of 10 to 80, preferably 30 to 70% by weight of styrene, 2 to 25, preferably 5 to 10% by weight of divinylbenzene, 1 to 88, preferably 20 to 50% by weight of a pore forming agent or of a solvent and also an active amount of a polymerization initiator, performance of the polymerization reaction with a temperature increase to 30 to 90°C and subsequent treatment with a sulphonating acid.

8. Supported catalyst according to one of Claims 1 to 6, obtainable by adding a mixture of (A) 10 to 80, preferably 20 to 50% by weight of styrene, 2 to 25, preferably 5 to 10% by weight of divinylbenzene, 1 to 88, preferably 20 to 50% by weight of a pore forming agent or solvent and also an active amount of a polymerization initiator and (B) a C₁₄- to C₁₇-n-paraffin fraction in a (A):(B) weight ratio of 10:1 to 1:10 to 5 to 50% by weight of a support material component relative to the total mixture, conditioning under vacuum, allowing to polymerize completely, washing-out of excess pore forming agent and of externally adhering polymer gel and subsequent treatment with a sulphonating acid.

9. Supported catalyst according to one of Claims 7 or 8, characterized in that the styrene is partially replaced by fluorostyrene.

10. Supported catalyst according to one of Claims 1 to 9, characterized in that the pore forming agent is a C₆-to C₁₆-alkane, such as n-heptane, pentadecane, isooctane or an n-paraffin fraction mainly containing C₉ to C₁₃.

11. Supported catalyst according to one of Claims 1 to 10, characterized in that the solvent is methanol, isooctane, pentadecane or a C₁₄- to C₁₇-n-paraffin cut.

12. Supported catalyst according to one of Claims 1 to 11, characterized in that the functionalization is carried out by means of a sulphonating acid, preferably an aromatic or aliphatic sulphonic acid, chlorosulphonic acid or sulphuric acid.

13. Supported catalyst according to Claim 12, characterized in that the aromatic sulphonic acid is benzenesulphonic acid.

14. Supported catalyst according to Claim 12, characterized in that the aliphatic sulphonic acid is methylsulphonic acid.

15. Use of a supported catalyst according to one of Claims 1 to 14 for performing the chemical reaction of etherification, esterification, hydration, dimerization, hydrogenation, alkylation, oligomerization or combinations of the same.

16. Use according to Claim 15, characterized by simultaneously applied separating operations such as adsorption, absorption, extraction, stripping, distillation, rectification, fractionation or diaphragm processes.

17. Use according to Claim 15 or 16, characterized by countercurrent feed of gas phase and liquid phase.

18. Use according to one of Claims 15 to 17, characterized by the reaction of tert-alkanols to form alkyl ethers.

19. Use according to one of Claims 15 to 18, characterized by the reaction of methanol and isobutene to form methyl tert-butyl ether (MTBE).

20. Use according to one of Claims 15 to 18, characterized by the reaction of isopropanol and isobutene to form isopropyl tert-butyl ether (PTBE).

21. Use according to one of Claims 15 to 18, characterized by the reaction of isobutene and ethanol to form ethyl tert-butyl ether (ETBE).

22. Use according to one of Claims 15 to 18, characterized by the reaction of 1-isopentene or 2-isopentene with methanol to form tert-amyl methyl ether (TAME).

23. Use according to one of Claims 15 to 17, characterized by the reaction of propene and water to form isopropanol.

24. Use according to one of Claims 15 to 17, characterized by the reaction of isobutene and water to form tert-butyl alcohol (TBA).

## Revendications

1. Catalyseur à support sous forme de corps de charge et constitué d'un matériau support à pores ouverts, où est ancré à sa surface interne et à sa surface externe, ainsi que dans le volume des pores mécaniquement et/ou chimiquement une résine échangeuse d'ions présentant des macropores, qu'on peut préparer par imprégnation ou couverture totale du matériau support en forme de corps de charge, par un mélange de monomères polymérisables et de solvants et/ou de formateurs de pores, réalisation de la réaction de polymérisation et le cas échéant fonctionnalisation par introduction de groupes actifs d'échange ionique.

2. Catalyseur à support selon la revendication 1,
caractérisé en ce que
les corps de charge sont en forme d'anneaux de Raschig, de selles de Berl, de selles toriques, d'anneaux complets avec nervure ou croisillon, d'anneaux Pall, d'autres corps creux, de billes creuses, d'empilements ordonnés, de nids d'abeille avec une teneur de l'espace vide de la résine d'échange ionique macroporeux de 5 à 95 % en volume.

3. Catalyseur à support selon la revendication 1 ou 2,
caractérisé en ce que
le matériau support est constitué de verre à pores ouverts, de verre fritté, de matériau céramique et pores ouverts à base de silicate d'aluminium, de céramique vitreuse frittée, de céramique expansée du charbon actif ou du coke actif.

4. Catalyseur à support selon l'une des revendications 1 à 3,
caractérisé en ce qu'
on fait réagir le matériau support à pores ouverts partant des groupes OH à sa surface avec un silane portant des groupes vinyle comme coupleur sur lequel on fixe des groupes échangeurs ioniques acides après sulfonation ultérieure.

5. Catalyseur à support selon l'une des revendications 1 à 4,
caractérisé en ce que
la résine échangeuse ionique macroporeuse est un poly(acide styrènesulfonique) réticulé macroporeux.

6. Catalyseur à support selon l'une des revendications 1 à 5, dopé par des métaux du 7ème ou aussi du 8ème sous-groupe du tableau périodique, notamment du palladium, du platine, du ruthénium, du rhodium, en des quantités de 0,1 à 100 g/kg de résine échangeuse ionique.

7. Catalyseur à support selon l'une des revendications 1 à 6,
caractérisé en ce qu'
on peut préparer par imprégnation du corps de charge avec 0,1 à 60 % en poids d'un mélange de 10 à 80, de préférence 30 à 70 % en poids de styrène, 2 à 25, de préférence 5 à 10 % en poids de divinylbenzène, 1 à 88, de préférence 20 à 50 % en poids d'un formateur de pore ou d'un solvant ainsi que d'une quantité efficace d'un initiateur de polymérisation, réalisation de la réaction de polymérisation par augmentation de température de 30 à 90°C et ensuite traitement par un acide de sulfonation.

8. Catalyseur à support selon l'une des revendications 1 à 6, qu'on peut préparer par addition d'un mélange (A) de 10 à 80, de préférence 20 à 50 % en poids de styrène, 2 à 25, de préférence 5 à 10 % en poids de divinylbenzène, 1 à 88 % de préférence 20 à 50 % en poids d'un formateur de pores ou d'un solvant ainsi qu'une quantité efficace d'un initiateur de polymérisation et (B) d'une fraction de n-paraffines C₁₄ à C₁₇ en rapport pondéral (A) : (B) de 10:1 à 1:10 pour 5 à 50 % en poids d'une teneur rapportée au mélange global du matériau support, traitement sous vide, polymérisation, traitement par lavage du formateur de pores en excès et du gel de polymère adhérent à l'extérieur et enfin traitement par un acide de sulfonation.

9. Catalyseur à support selon l'une des revendications 7 ou 8,
caractérisé en ce qu'on remplace en partie le styrène par du fluorostyrène.

10. Catalyseur à support selon l'une des revendications 1 à 9,
caractérisé en ce que
comme formateur de pores on utilise des alcanes C₆ à C₁₆, comme les n-heptane, pentadécane, i-octane, ainsi qu'une fraction C₉ à C₁₃ des n-paraffines.

11. Catalyseur à support selon l'une des revendications 1 à 10,
caractérisé en ce que
le solvant est du méthanol, de l'i-octane, du pentadécane ou une coupe de n-paraffines C₁₄ à C₁₇.

12. Catalyseur à support selon l'une des revendications 1 à 11,
caractérisé en ce qu'
on réalise la fonctionnalisation au moyen d'un acide de sulfonation, de préférence un acide sulfonique aromatique ou aliphatique, l'acide chlorosulfonique ou l'acide sulfonique.

13. Catalyseur à support selon la revendication 12,
caractérisé en ce que
l'acide sulfonique aromatique est l'acide benzène sulfonique.

14. Catalyseur à support selon la revendication 12,
caractérisé en ce que
l'acide sulfonique aliphatique est l'acide méthylsulfonique.

15. Utilisation d'un catalyseur à support selon l'une des revendications 1 à 14 pour réaliser la réaction chimique de l'éthérification, de l'estérification, de l'hydrogénation, de l'akylation, de l'hydratation, de la dimérisation, de l'oligomérisation ou de leurs combinaisons.

16. Utilisation selon la revendication 15,
caractérisée par
les opérations appliquées simultanées de séparation comme l'adsorption, l'adsorption, l'extraction, le stripage, la distillation, la rectification, le fractionnement ou les procédés de membrane.

17. Utilisation selon la revendication 15 ou 16,
caractérisée par
l'introduction à contre-courant de la phase gazeuse et de la phase liquide.

18. Utilisation selon l'une des revendications 15 à 17,
caractérisée par
la transformation d'alcanols en éthers ter-alkyliques.

19. Utilisation selon l'une des revendications 15 à 18,
caractérisée par
la transformation de méthanol et d'i-butène en éther méthyl-tert-butylique (MTBE).

20. Utilisation selon l'une des revendications 15 à 18,
caractérisée par
la transformation d'i-propanol et d'i-butène en éther i-propyl-tert-butylique (PTBE).

21. Utilisation selon l'une des revendications 15 à 18,
caractérisée par
la transformation d'i-butène et d'éthanol en éther éthyl-tert-butylique (ETBE).

22. Utilisation selon l'une des revendications 15 à 18,
caractérisée par
la transformation d'i-pentène-(1) ou d'i-pentène-(2) avec du méthanol pour donner l'éther tert-amylméthylique (TAME).

23. Utilisation selon l'une des revendications 15 à 17,
caractérisée par
la transformation de propène et d'eau en i-propanol.

24. Utilisation selon l'une des revendications 15 à 17,
caractérisée par
la transformation d'i-butène et d'eau en tert-butanol (TBA).
